(19) Europäisches Patentamt
European Patent Office
Office européen des brevets

(11) EP 1 283 854 B1

(12) # EUROPÄISCHE PATENTSCHRIFT

(45) Veröffentlichungstag und Bekanntmachung des Hinweises auf die Patenterteilung:
**17.11.2004 Patentblatt 2004/47**

(21) Anmeldenummer: **01936361.3**

(22) Anmeldetag: **15.05.2001**

(51) Int Cl.7: **C08L 71/02**, A61K 7/06, A61K 7/48

(86) Internationale Anmeldenummer:
**PCT/EP2001/005501**

(87) Internationale Veröffentlichungsnummer:
**WO 2001/090245 (29.11.2001 Gazette 2001/48)**

(54) **LÖSUNGSVERMITTLER**
SOLUTIZING AGENTS
AGENT DE SOLUBILISATION

(84) Benannte Vertragsstaaten:
**DE ES FR GB IT**

(30) Priorität: **25.05.2000 DE 10025756**

(43) Veröffentlichungstag der Anmeldung:
**19.02.2003 Patentblatt 2003/08**

(73) Patentinhaber: **Cognis Deutschland GmbH & Co. KG**
**40589 Düsseldorf (DE)**

(72) Erfinder:
- **CORBELLA, Alberto**
**I-22071 Como (IT)**
- **SOMIGLIANA, Christian**
**22020 Torno (IT)**

(56) Entgegenhaltungen:
**DE-A- 19 824 359** **US-A- 4 155 936**
**US-A- 4 832 868**

- **PATENT ABSTRACTS OF JAPAN vol. 1999, no. 11, 30. September 1999 (1999-09-30) & JP 11 148091 A (KAO CORP), 2. Juni 1999 (1999-06-02)**
- **PATENT ABSTRACTS OF JAPAN vol. 1999, no. 03, 31. März 1999 (1999-03-31) & JP 10 324892 A (DAI ICHI KOGYO SEIYAKU CO LTD), 8. Dezember 1998 (1998-12-08)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 283 854 B1

## Beschreibung

### Gebiet der Erfindung

Die Erfindung befindet sich auf dem Gebiet der Kosmetik und betrifft neue Lösungsvermittler mit einem erhöhten Lösungsvermögen insbesondere für Parfümöle und UV-Lichtschutzfilter.

### Stand der Technik

Lipophile Stoffe, wie beispielsweise Vitamine, Parfümöle oder UV-Lichtscchutzfilter lassen sich vielfach nur schwer in kosmetische oder pharmazeutische Zubereitungen einarbeiten, insbesondere dann, wenn diese einen überwiegend polaren Charakter aufweisen. In solchen Fällen kommen Lösungsvermittler zum Einsatz, bei denen es sich um einzelne Stoffe oder Mischungen mit mittleren HLB-Werten handelt, die also gewissermaßen eine Brücke von der polaren Umgebung zum unpolaren Substrat bilden. Sehr effektive Hydrotrope stellen die Sulfonate kurzkettiger Alkylaromaten, wie z.B. Toluol- oder Cumolsulfonat dar, die wegen ihrer unzureichenden hautkosmetischen Verträglichkeit aber im Bereich der Kosmetik keine Bedeutung haben. Andere kosmetische Solubilisatoren, wie z.B. spezielle hydrophilisierte Öle, sind zwar hautverträglich, besitzen aber kein ausreichendes Lösungsvermögen und/oder ein schlechtes Kälteverhalten, d.h. zeigen schon bei Raumtemperatur die Tendenz zur Austrübung. Aus diesem Grunde besteht vor allem in der kosmetischen Industrie der Wunsch nach neuen Lösungsvermittlern, die frei von den oben geschilderten Nachteilen sind.

Die Aufgabe der vorliegenden Erfindung hat somit darin bestanden, neue Lösungsvermittler zur Verfügung zu stellen, die gegenüber den Produkten des Stands der Technik ein verbessertes Lösungsvermögen insbesondere gegenüber lipophilen Stoffen, wie z.B. Parfümölen, Vitaminen, UV-Uchtschutzfilter und dergleichen aufweisen, dabei bei Raumtemperatur flüssig sind und einen Kältetrübungspunkt unterhalb von 10 °C aufweisen.

### Beschreibung der Erfindung

Gegenstand der Erfindung sind Lösungsvermittler, enthaltend

(a) Anlagerungsprodukte von Ethylenoxid an Fettalkohole,
(b) Anlagerungsprodukte von Ethylenoxid und Propylenoxid an Fettalkohole, und
(c) Anlagerungsprodukte von Ethylenoxid an Triglyceride.

Überraschenderweise wurde gefunden, dass Zubereitungen der genannten Art gegenüber Produkten des Stands der Technik ein deutlich verbessertes Lösungsvermögen insbesondere gegenüber Parfümölen, Vitaminen und UV-Lichtschutzfiltern aufweisen. Ein weiterer Vorteil besteht darin, dass die Mischungen bei Raumtemperatur flüssig sind, sich daher leicht verarbeiten lassen und zudem einen Kältetrübungspunkt im gewünschten Bereich, nämlich unter 10 °C aufweisen.

Anlagerungsprodukte von Ethylenoxid an Fettalkohole

Bei den Anlagerungsprodukten von Ethylenoxid an Fettalkohole, die als Komponente (a) in Frage kommen, handelt es sich um bekannten nichtionische Tenside vom Typ der Alkoholpolyethylenglycolether, die vorzugsweise der Formel (I) folgen,

$$R^1O(CH_2CH_2O)_nH \quad \text{(I)}$$

in der $R^1$ für einen linearen oder verzweigten Alkyl- und/oder Alkenylrest mit 6 bis 22, vorzugsweise 12 bis 18 Kohlenstoffatomen und n für Zahlen von 1 bis 20 steht. Die Herstellung der Produkte erfolgt großtechnisch durch basenkatalysierte Anlagerung von Ethylenoxid an die primäre Hydroxylfunktion der Alkohole, wobei in Abhängigkeit des gewählten Katalysators Produkte mit einer konventionell breiten oder aber eingeschränkten Homologenverbeilung resultieren. Typische beispiele sind die Anlagerungsprodukte von durchschnittlich 1 bis 20 und vorzugsweise 5 bis 10 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt ist der Einsatz von Anlagerungsprodukten von durchschnittlich 5 bis 10 Mol Ethylenoxid an Fettalkohole mit 12 bis 18 Kohlenstoffatome.

Anlagerungsprodukte von Ethylen- und Propylenoxid an Fettalkohole

Bei den Anlagerungsprodukten von Ethylen- und Propylenoxid an Fettalkohole, welche die Komponente (b) bilden, handelt es sich um analoge Verbindungen zu den Stoffen der Gruppe (a), die jedoch mindestens eine Propylenoxideinheit in der Polyetherkette aufweisen. Die Fettalkoholpolyethylen/polypropylenglycolether folgen vorzugsweise Formel **(II),**

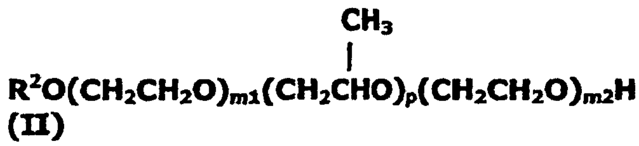

**(II)**

in der $R^2$ für einen linearen oder verzweigten Alkyl- und/oder Alkenylrest mit 6 bis 22, vorzugsweise 12 bis 18 Kohlenstoffatomen, p für Zahlen von 1 bis 10, vorzugsweise 1 bis 2 sowie m1 und m2 unabhängig voneinander für Zahlen von 0 bis 20, vorzugsweise 2 bis 15 stehen, mit der Maßgabe, dass die Summe (m1+m2) ungleich 0 ist. Typische Beispiele sind die Anlagerungsprodukte von durchschnittlich 1 bis 20 Mol Ethylenoxid und 1 bis 10 Mol Propylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkahol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Bezogen auf die Polyetherkette können die Ethylen- und Propylenoxideinheiten dabei in Block- oder Randomverteilung vorliegen; vorzugsweise bilden Ethylenoxideinheiten der Abschluß der Polyetherkette. Besonders bevorzugt sind Anlagerungsprodukte von durchschnittlich 5 bis 10 Mol Ethylenoxid und 1 bis 2 Mol Propylenoxid an Fettalkohole mit 12 bis 18 Kohlenstoffatome.

Anlagerungsprodukte von Ethylenoxid an Triglyceride

Auch bei den Anlagerungsprodukten von Ethylenoxid an Triglyceride, die die Komponente (c) bilden, handelt es sich um bekannte nichtionische Tenside. In Frage kommen beispielsweise Anlagerungsprodukte von durchschnittlich 20 bis 100 und vorzugsweise 30 bis 50 Mol Ethylenoxid an Triglyceride, deren Acylreste sich von Fettsäuren mit 6 bis 22, vorzugsweise 12 bis 18 Kohlenstoffatomen ableiten. Die Triglyceride können synthetischer Natur sein, vorzugsweise handelt es sich jedoch um natürliche, speziell pflanzliche Fette und Öle, die nach Raffination und gegebenenfalls Härtung mit Ethylenoxid umgesetzt werden. Die Ethoxylierung kann dabei durch Einschub von Ethylenoxid in die Carbonylestergruppe erfolgen, leichter und daher auch bevorzugt ist jedoch die Anlagerung an im Molekül vorhandene sekundäre Hydroxylgruppen, weshalb Ricinusöl (Castoröl) sowie dessen Härtungsprodukt als Ausgangsstoffe besonders bevorzugt sind. Aus anwendungstechnischer Sicht empfiehlt sich insbesondere der Einsatz von Anlagerungsprodukten von durchschnittlich 30 bis 50 Mol Ethylenoxid an Castoröl oder dessen Härtungsprodukt.

Lösungsvermittler

In einer bevorzugten Ausführungsform der vorliegenden Erfindung enthalten die Lösungsvermittler

(a) 30 bis 50, vorzugsweise 35 bis 45 Gew.-% Anlagerungsprodukte von Ethylenoxid an Fettalkohole,
(b) 20 bis 40, vorzugsweise 25 bis 35 Gew.-% Anlagerungsprodukte von Ethylenoxid und Propylenoxid an Fettalkohole, und
(c) 10 bis 30, 15 bis 25 Gew.-% Anlagerungsprodukte von Ethylenoxid an Triglyceride

mit der Maßgabe, dass sich die Mengenangaben gegebenenfalls zusammen mit Wasser zu 100 Gew.-% ergänzen. Der Wassergehalt der Mischungen liegt üblicherweise im Bereich von 1 bis 20, vorzugsweise 5 bis 15 Gew.-%.

## Gewerbliche Anwendbarkeit

Ein weiterer Gegenstand der Erfindung betrifft die Verwendung von Mischungen, enthaltend

(a) Anlagerungsprodukte von Ethylenoxid an Fettalkohole,
(b) Anlagerungsprodukte von Ethylenoxid und Propylenoxid an Fettalkohole, und
(c) Anlagerungsprodukte von Ethylenoxid an Triglyceride

als Lösungsvermittler zur Herstellung von kosmetischen und/oder pharmazeutischen Zubereitungen, in denen sie in Mengen von 1 bis 20, vorzugsweise 3 bis 15 und insbesondere 5 bis 10 Gew.-% enthalten sein können. Gegenstand der Erfindung sind auch kosmetische Zusammensetzung enthaltend 1 bis 20 Gew.-% eines Lösungsvermittlers mit einem Gehalt an

(a) 30 bis 50 Gew.-% Anlagerungsprodukte von Ethylenoxid an Fettalkohole,
(b) 20 bis 40 Gew.-% Anlagerungsprodukte von Ethylenoxid und Propylenoxid an Fett-alkohole, und
(c) 10 bis 30 Gew.-% Anlagerungsprodukte von Ethylenoxid an Triglyceride.

### Kosmetische und/oder pharmazeutische Zubereitungen

Die erfindungsgemäßen Lösungsvermittler können zur Herstellung von kosmetischen und/oder pharmazeutischen Zubereitungen, wie beispielsweise Haarshampoos, Haarlotionen, Schaumbäder, Duschbäder, Mund- und Zahnpflegemittel, Cremes, Gele, Lotionen, alkoholische und wäßrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Uchtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Co-Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, $\alpha$-Methylestersulfonate, Sulfofett-säuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fetkalkoholpolyglycolether, Alkylphenolpolyglycolether, Fettsäurepolyglycol-ester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, $\alpha$-Olefinsulfonate, Ethercarbonsäuren, Alkyloligoglucoside, Fettsäureglucamide, Alkylamidobetaine, Amphoacetate

und/oder Proteinfettsäurekonden-sate, letztere vorzugsweise auf Basis von Weizenproteinen.

Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen oder verzweigten $C_6$-$C_{22}$-Fettalkoholen bzw. Ester von verzweigten $C_6$-$C_{13}$-Carbonsäuren mit linearen oder verzweigten $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpatmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von $C_{18}$-$C_{38}$-Alkylhydroxycarbonsäuren mit linearen oder verzweigten $C_6$-$C_{22}$-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren; Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestem mit Polyolen, Siliconöle (Cydomethicone, Siliciummethicontypen u.a.) und/oder aliphatische bzw. naphthenische Kohlenwasserstoffe, wie z.B. wie Squalan, Squalen oder Dialkylcyclohexane in Betracht.

Emulgatoren

Als Emulgatoren kommen beispielsweise nichtionogene Tenside aus mindestens einer der folgenden Gruppen in Frage:

- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie

- Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht $C_{12/18}$-Fettsäuremono- und -diester von Anlagerungsprodukten von Ethylenoxid an Glycerin sind aus **DE 2024051 PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cydischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxysbearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sarbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammonlumglycinate, beispielsweise das Kokosacylaminopropyldimethylammoniumglyclnat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12/18}$-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Waltat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Stabilisatoren

Als Stabilisatoren können Metallsalze von Fettsäuren, wie z.B. Magnesium-, Aluminium- und/oder Zinkstearat bzw. -ricinoleat eingesetzt werden.

Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie

z.B. Luviquat® (BASF), Kondensationsprodukte von Polyglycolen und Aminen, quatemierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapoi® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobomylacrylat-Copolymere, Methylvinylether/Maleinsäureanhydrid-Copolymere und deren Ester, unvemetzte und mit Potyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/ Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosmetics & Toiletries Vol. 108, Mai 1993, Seite 95ff** aufgeführt.

<u>Siliconverbindungen</u>

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. <u>91</u>, 27 (1976).**

<u>UV-Lichtschutzfilter und Antioxidantien</u>

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:

- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure

und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans z.B. 4-tert-Butyl-4'-methoxydibenzoylmethan (Parsol 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Uchtschutzfilter sind der Übersicht von P.Finkel in **SÖFW-Journal <u>122</u>, 543 (1996)** sowie **Parf.Kosm. <u>3</u>, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beisplele hierfür sind Aminosäuren (z.B. Glycin, Histidin, Tyrosin, Tryptophan) und deren Derivate, Imidazole (z.B. Urocaninsäure) und deren Derivate, Peptide wie D, L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

<u>Biogene Wirkstoffe</u>

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)-harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutyl-carbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet Hierbei handelt es sich vorzugsweise um Trialkylcitrate wie Trimethylcitrat, Tripropylcitrat, Trüsopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riedistoffverbindungen vom Typ der Ester sind z.B. Benzylacetat, p-tert.-Butylcydohexylacetat, Linalylacetat, Phenyl ethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:

- adstringierende Wirkstoffe,
- Ölkomponenten,
- nichtionische Emulgatoren,
- Coemulgatoren,
- Konsistenzgeber,

- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:

- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Alurniniumpyrithion und Magnesiumpyrithion / Dipyrithion-Magnesiumsulfat in Frage.

Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R. Lochhead in **Cosm.Toil. 108, 95 (1993)** entnommen werden.

Insekten-Repellentien

Als Insekten-Repellentien kommen N,N-Diethyl-m-toluamid, 1,2-Pentandiol oder Ethyl Butylacetylaminopropionate in Frage

Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cydamenaldehyd, Hydroxycitronellal, Lilial und Bourgeonal, zu den Ketonen z.B. die Jonone, $\alpha$-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cydovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

**Beispiele**

Zur Überprüfung des Lösungsvermögen wurden 10 g Lösungsvermittler solange mit verschiedenen Testsubstanzen versetzt, bis die Löslichkeitsgrenze erreicht wurde. Die Ergebnisse sind in Tabelle 1 zusammengefaßt. Beispiel 1 ist erfindungsgemäß, die Beispiele V1 und V2 dienen zum Vergleich.

**Tabelle 1**
**Lösungsvermögen (Mengenangaben als Gew.-%)**

| Zusammensetzung / Löslichkeit | 1 | V1 | V2 |
|---|---|---|---|
| Cocoeth-8 | 40 | - | 100 |
| PPG1-PEG9 Lauryl Glycol Ether | 30 | - | - |
| PEG-40 Hydrogenated Castor Oil | 20 | 100 | - |
| Wasser | 10 | - | - |
| ***Löslichkeit [g/10 g Lösungsvermittler]*** | | | |
| Citrus Bergamia | 3,15 | 1,00 | 0,90 |
| Citrus Sinensis | 5,88 | 0,65 | 0,55 |
| Citrus Limonum | 1,70 | 0,70 | 0,71 |
| Menthol | 4,80 | 1,10 | 1,05 |
| Thymus | 1,60 | 0,66 | 0,68 |
| Triticum Vulgare | 0,90 | 0,05 | 0,04 |
| Geranium | 12,5 | 1,12 | 1,10 |
| Mentha | 7,14 | 1,40 | 1,20 |
| Tocopheryl Acetate | 3,85 | 0,66 | 0,70 |
| Tocopherol | 0,90 | 0,40 | 0,50 |
| Persea Gratissima | 0,72 | < 0,05 | < 0,05 |
| Ricinus Communis | 0,42 | < 0,05 | < 0,05 |
| Placentalipo | 11,1 | | |
| Zantzix Oil | 9,00 | 1,12 | 0,90 |
| Tea Tree Oil | 2,80 | 0,95 | 0,90 |
| Citronella | 1,20 | 0,22 | 0,30 |
| Benzophenone-3 | 0,44 | 0,40 | 0,20 |
| Octyl Methoxycinnamate | 1,60 | 0,45 | 0,60 |
| Isoamyl Methoxycinnamate | 1,10 | 0,60 | 0,70 |
| 4-Methylbenzylidene Camphor | 1,10 | 0,50 | 0,38 |

**Patentansprüche**

**1.** Lösungsvermittler, enthaltend

(a) Anlagerungsprodukte von Ethylenoxid an Fettalkohole,
(b) Anlagerungsprodukte von Ethylenoxid und Propylenoxid an Fettalkohole, und
(c) Anlagerungsprodukte von Ethylenoxid an Triglyceride.

2. Lösungsvermittler nach Anspruch 1, **dadurch gekennzeichnet, dass** sie als Komponente (a) Anlagerungsprodukte von Ethylenoxid an Fettalkohole der Formel (I) enthalten,

$$R^1O(CH_2CH_2O)_nH$$
**(I)**

in der $R^1$ für einen linearen oder verzweigten Alkyl- und/oder Alkenylrest mit 6 bis 22 Kohlenstoffatomen und n für Zahlen von 1 bis 20 steht.

3. Lösungsvermittler nach den Ansprüchen 1 und/oder 2, **dadurch gekennzeichnet, dass** sie als Komponente (a) Anlagerungsprodukte von durchschnittlich 5 bis 10 Mol Ethylenoxid an Fettalkohole mit 12 bis 18 Kohlenstoffatome enthalten.

4. Lösungsvermittler nach mindestens einem der Ansprüche 1 bis 3, **dadurch gekennzeichnet, dass** sie als Komponente (b) Anlagerungsprodukte von Ethylenoxid und Propylenoxid an Fettalkohole der Formel **(II)** enthalten,

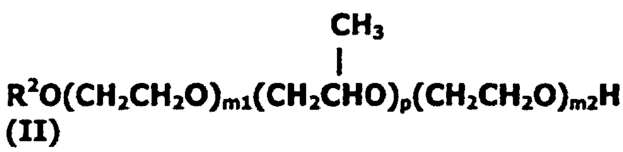
**(II)**

in der $R^2$ für einen linearen oder verzweigten Alkyl- und/oder Alkenylrest mit 6 bis 22 Kohlenstoffatomen, p für Zahlen von 1 bis 10 sowie m1 und m2 unabhängig voneinander für Zahlen von 0 bis 20 stehen, mit der Maßgabe, dass die Summe (m1+m2) ungleich 0 ist.

5. Lösungsvermittler nach mindestens einem der Ansprüche 1 bis 4, **dadurch gekennzeichnet, dass** sie als Komponente (b) Anlagerungsprodukte von durchschnittlich 5 bis 10 Mol Ethylenoxid und 1 bis 2 Mol Propylenoxid an Fettalkohole mit 12 bis 18 Kohlenstoffatome enthalten.

6. Lösungsvermittler nach mindestens einem der Ansprüche 1 bis 5, **dadurch gekennzeichnet, dass** sie als Komponente (c) Anlagerungsprodukte von durchschnittlich 20 bis 100 Mol Ethylenoxid an Triglyceride enthalten, deren Acylreste sich von Fettsäuren mit 6 bis 22 Kohlenstoffatomen ableiten.

7. Lösungsvermittler nach mindestens einem der Ansprüche 1 bis 6, **dadurch gekennzeichnet, dass** sie als Komponente (c) Anlagerungsprodukte von durchschnittlich 30 bis 50 Mol Ethylenoxid an Castoröl oder dessen Härtungsprodukt enthalten.

8. Lösungsvermittler nach mindestens einem der Ansprüche 1 bis 7, **dadurch gekennzeichnet, dass** sie

(a) 30 bis 50 Gew.-% Anlagerungsprodukte von Ethylenoxid an Fettalkohole,
(b) 20 bis 40 Gew.-% Anlagerungsprodukte von Ethylenoxid und Propylenoxid an Fettalkohole, und
(c) 10 bis 30 Gew.-% Anlagerungsprodukte von Ethylenoxid an Triglyceride

mit der Maßgabe enthalten, dass sich die Mengenangaben gegebenenfalls zusammen mit Wasser zu 100 Gew.-% ergänzen.

9. Lösungsvermittler nach mindestens einem der Ansprüche 1 bis 8, **dadurch gekennzeichnet, dass** die Lösungsvermittler 1 bis 20 Gew.-% Wasser enthalten.

10. Verwendung von Mischungen, enthaltend

(a) Anlagerungsprodukte von Ethylenoxid an Fettalkohole,
(b) Anlagerungsprodukte von Ethylenoxid und Propylenoxid an Fettalkohole, und

(c) Anlagerungsprodukte von Ethylenoxid an Triglyceride

als Lösungsvermittler zur Herstellung von kosmetischen und/oder pharmazeutischen Zubereitungen.

**11.** Kosmetische Zusammensetzung enthaltend 1 bis 20 Gew.-% eines Lösungsvermittlers mit einem Gehalt an:

(a) 30 bis 50 Gew.-% Anlagerungsprodukte von Ethylenoxid an Fettalkohole,
(b) 20 bis 40 Gew.-% Anlagerungsprodukte von Ethylenoxid und Propylenoxid an Fettalkohole, und
(c) 10 bis 30 Gew.-% Anlagerungsprodukte von Ethylenoxid an Triglyceride.

## Claims

**1.** Solubilizers containing

(a) products of the addition of ethylene oxide onto fatty alcohols,
(b) products of the addition of ethylene oxide and propylene oxide onto fatty alcohols and
(c) products of the addition of ethylene oxide onto triglycerides.

**2.** Solubilizers as claimed in claim 1, **characterized in that** they contain products of the addition of ethylene oxide onto fatty alcohols corresponding to formula **(I)**:

$$R^1O(CH_2CH_2O)_nH \qquad (I)$$

in which $R^1$ is a linear or branched alkyl and/or alkenyl group containing 6 to 22 carbon atoms and n is a number of 1 to 20,
as component (a).

**3.** Solubilizers as claimed in claims 1 and/or 2, **characterized in that** they contain products of the addition of on average 5 to 10 mol ethylene oxide onto $C_{12-18}$ fatty alcohols as component (a).

**4.** Solubilizers as claimed in at least one of claims 1 to 3, **characterized in that** they contain products of the addition of ethylene and propylene oxide onto fatty alcohols corresponding to formula **(II)**:

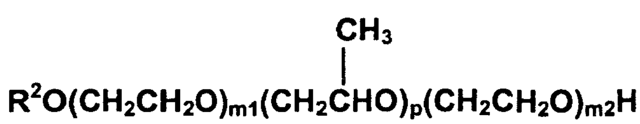

**(II)**

in which $R^2$ is a linear or branched alkyl and/or alkenyl group containing 6 to 22 carbon atoms, p is a number of 1 to 10 and m1 and m2 independently of one another stand for numbers of 0 to 20, with the proviso that the sum (m1+m2) is not 0, as component (b).

**5.** Solubilizers as claimed in at least one of claims 1 to 4, **characterized in that** they contain products of the addition of on average 10 mol ethylene oxide and 1 to 2 mol propylene oxide onto $C_{12-18}$ fatty alcohols as component (b).

**6.** Solubilizers as claimed in at least one of claims 1 to 5, **characterized in that** they contain products of the addition of on average 20 to 100 mol ethylene oxide onto triglycerides, of which the acyl groups are derived from $C_{6-22}$ fatty acids, as component (c).

**7.** Solubilizers as claimed in at least one of claims 1 to 6, **characterized in that** they contain products of the addition of on average 30 to 50 mol ethylene oxide onto castor oil or the hydrogenation product thereof as component (c).

8. Solubilizers as claimed in at least one of claims 1 to 7, **characterized in that** they contain

(a) 30 to 50% by weight of addition products of ethylene oxide onto fatty alcohols,
(b) 20 to 40% by weight of addition products of ethylene oxide and propylene oxide onto fatty alcohols and
(c) 10 to 30% by weight of addition products of ethylene oxide onto triglycerides,

with the proviso that the quantities shown add up to 100% by weight, optionally together with water.

9. Solubilizers as claimed in at least one of claims 1 to 8, **characterized in that** the solubilizers contain 1 to 20% by weight of water.

10. The use of mixtures containing

(a) products of the addition of ethylene oxide onto fatty alcohols,
(b) products of the addition of ethylene oxide and propylene oxide onto fatty alcohols and
(c) products of the addition of ethylene oxide onto triglycerides

as solubilizers for the production of cosmetic and/or pharmaceutical preparations.

11. A cosmetic composition containing 1 to 20% by weight of a solubilizer containing

(a) 30 to 50% by weight of addition products of ethylene oxide onto fatty alcohols,
(b) 20 to 40% by weight of addition products of ethylene oxide and propylene oxide onto fatty alcohols and
(c) 10 to 30% by weight of addition products of ethylene oxide onto triglycerides.

**Revendications**

1. Agents solubilisants contenant

a) des produits d'addition de l'oxyde d'éthylène sur des alcools gras,
b) des produits d'addition de l'oxyde d'éthylène et de l'oxyde de propylène sur des alcools gras et
c) des produits d'addition de l'oxyde d'éthylène sur des triglycérides.

2. Agents solubilisants selon la revendication 1,
**caractérisés en ce qu'**
ils renferment comme composant a) des produits d'addition de l'oxyde d'éthylène sur des alcools gras de formule (I)

$$R^1O(CH_2CH_2O)_nH \qquad (I)$$

dans laquelle $R^1$ représente un reste alkyle et/ou alkényle linéaire ou ramifié ayant de 6 à 22 atomes de carbone et n représente des nombres allant de 1 à 20.

3. Agents solubilisants selon les revendications 1 et/ou 2,
**caractérisés en ce qu'**
ils renferment comme composant a) des produits d'addition de - en moyenne - 5 à 10 mol. d'oxyde d'éthylène sur des alcools gras ayant de 12 à 18 atomes de carbone.

4. Agents solubilisants selon au moins une des revendications 1 à 3,
**caractérisés en ce qu'**
ils renferment comme composant b) des produits d'addition de l'oxyde d'éthylène et de l'oxyde de propylène sur des alcools gras de formule (II)

$$R^2O(CH_2CH_2O)_{m1}(CH_2\underset{\displaystyle CH_3}{\overset{|}{C}}HO)_p(CH_2CH_2O)_{m2}H \quad \text{(II)}$$

dans laquelle $R^2$ représente un reste alkyle et/ou alkényle linéaire ou ramifié, ayant de 6 à 22 atomes de carbone, p représente des nombres allant de 1 à 10 ainsi que $m_1$ et $m_2$ indépendamment l'un de l'autre représentent des nombres allant de 0 à 20, avec la précision que la somme $(m_1 + m_2)$ est différente de 0.

**5.** Agents solubilisants selon au moins l'une des revendications 1 à 4,
**caractérisés en ce qu'**
ils renferment comme composant b) des produits d'addition de - en moyenne - 5 à 10 mol. d'oxyde d'éthylène et de 1 à 2 mol. d'oxyde de propylène sur des alcools gras ayant de 12 à 18 atomes de carbone.

**6.** Agents solubilisants selon au moins l'une des revendications 1 à 5,
**caractérisés en ce qu'**
ils renferment comme composant c) des produits d'addition de - en moyenne - 20 à 100 mol. d'oxyde d'éthylène sur des triglycérides dont les restes acyle dérivent d'acides gras ayant de 6 à 22 atomes de carbone.

**7.** Agents solubilisants selon au moins l'une des revendications 1 à 6,
**caractérisés en ce qu'**
ils renferment comme composant c) des produits d'addition de - en moyenne - 30 à 50 mol. d'oxyde d'éthylène sur de l'huile de ricin ou de son produit de durcissement.

**8.** Agents solubilisants selon au moins l'une des revendications 1 à 7,
**caractérisés en ce qu'**
ils renferment

a) de 30 à 50 % en poids de produits d'addition de l'oxyde d'éthylène sur des alcools gras,
b) de 20 à 40 % en poids de produits d'addition d'oxyde d'éthylène et d'oxyde de propylène sur des alcools gras,
c) de 10 à 30 % en poids de produits d'addition d'oxyde d'éthylène sur des triglycérides,

avec la précision que les données en quantités se complètent, le cas échéant avec de l'eau, à 100 % en poids.

**9.** Agents solubilisants selon au moins l'une des revendications 1 à 8,
**caractérisés en ce que**
les agents solubilisants contiennent de 1 à 20 % en poids d'eau.

**10.** Utilisation de mélanges contenant

a) des produits d'addition de l'oxyde d'éthylène sur des alcools gras,
b) des produits d'addition de l'oxyde d'éthylène et de l'oxyde de propylène sur des alcools gras et
c) des produits d'addition de l'oxyde d'éthylène sur des triglycérides,

comme agents solubilisants en vue de la production de préparations cosmétiques et/ou pharmaceutiques.

**11.** Composition cosmétique contenant de 1 à 20 % en poids d'un agent solubilisant ayant une teneur en :

a) de 30 à 50 % en poids de produits d'addition de l'oxyde d'éthylène sur des alcools gras,
b) de 20 à 40 % en poids de produits d'addition de l'oxyde d'éthylène et de l'oxyde de propylène sur des alcools gras,
c) de 10 à 30 % en poids de produits d'addition de l'oxyde d'éthylène sur des triglycérides.